# EUROPEAN PATENT SPECIFICATION

(11) **EP 0 368 043 B1**
(45) Date of publication and mention of the grant of the patent: **22.02.1995**
(21) Application number: 89119342.7
(22) Date of filing: 18.10.1989
(51) Int. Cl.: A61B 17/12

(54) **Disposable tourniquet**
Einweg-Aderpresse
Tourniquet à usage unique

(30) Priority: 19.10.1988 IT 2204088 U
(43) Date of publication of application: 16.05.1990
(73) Proprietor: Dioguardi, Francesco Saverio, I-20121 Milan (IT)
(72) Inventor: Dioguardi, Francesco Saverio, I-20121 Milan (IT)
(74) Representative: Gervasi, Gemma, Dr.

(56) References cited:
- FR-A- 441 625
- US-A- 3 670 735
- US-A- 3 930 506

## Description

This invention relates to a tourniquet consisting of a fibrous material structure with an elasticity equivalent to the one of traditional rubber tourniquets but very much cheaper to produce and therefore suitable for single use.

The tourniquet, according to the present invention, shows highly useful characteristics, as will be shown in the following detailed description.

The spread of highly infectious viral diseases has accentuated the need for adequate preventive measures in regard to sanitary materials.

For example, disposable syringes are generally used by health services and by individuals instead of glass ones.

This provides maximum guarantee against infections along with maximal practicality. In fact, it is not economic to sterilize syringes, since sterilization costs more than the syringe itself.

Similar statements can be made for bandages, gauzes, tapes and bandaids, once recycled after sterilization and now thrown away after one use.

The decisive factor in this development was their manufacture in paper, thanks to the mechanical strength (especially stretch resistance) of this material and thanks to the ease of disposing of them by incineration without polluting the environment.

For these reasons, smocks, masks, hats, drapes and sheets in operating room are now made of cellulose fibre materials.

In addition to this, much attention is given now to protecting the medical and paramedical personnel from accidental injection by infectious blood and there are continuous public campaigns not to use syringes previously used by others.

However, no attention has been paid to the tourniquet, which is indispensable to anyone who has to give an intravenous injection.

There are several kinds of tourniquet. The most widely used is the traditional rubber tube used for many patients in succession and over long periods of time.

Other types of tourniquets have been proposed as disclosed for example in the following patents.

French Patent n. 0 441 625 discloses a tourniquet based on a metallic spiral spring. US Patent n. 3,930,506 discloses a tourniquet comprising a flat elastic band of plasticized polyvinylchloride. US Patent n. 3,670,735 discloses a tourniquet which includes an inflatable envelope formed from three layers of vinyl plastic.

None of said tourniquets has the characteristics of a cheap disposable tourniquet which can be destroyed by incineration without polluting the atmosphere.

Since the appearance of the acquired immuno-deficiency syndrome (AIDS), a great deal of attention has been paid to the risk of transmission of the infection from patient to patient or from patient to persons treating him, or viceversa.

Therefore, it should be helpful to have available a disposable tourniquet to avoid even that possibility of contagion and which, in addition, can be destroyed by incineration without polluting the atmosphere.

This object has been achieved with the tourniquet according to the invention, which consists of a tube made of a diagonally woven fibrous mesh, and, containing an elastic component.

These and other characteristics and advantages of the tourniquet, according to the present invention, will be made more evident by the following description illustrated by the enclosed figures.

Figures 1,2 and 4 indicate three ways to prepare the tourniquet, Figure 1 with no elastic component, Figure 2 with peripheral elastic system and Figure 4 with a central elastic system.

Figure 3 shows a transverse section of the tourniquet of Figure 2.

Figures 5 and 6 are two different embodiments of the tubular fabric component and Figure 7 shows the application of the tourniquet to the arm.

Referring to the reference numerals in the various figures: 1 is the fibre material fabric which can be vegetable, synthetic or mixed.

This fabric is woven diagonally and can consist for example of interwoven threads or tapes.

Preferably the fibrous material is cellulose paper.

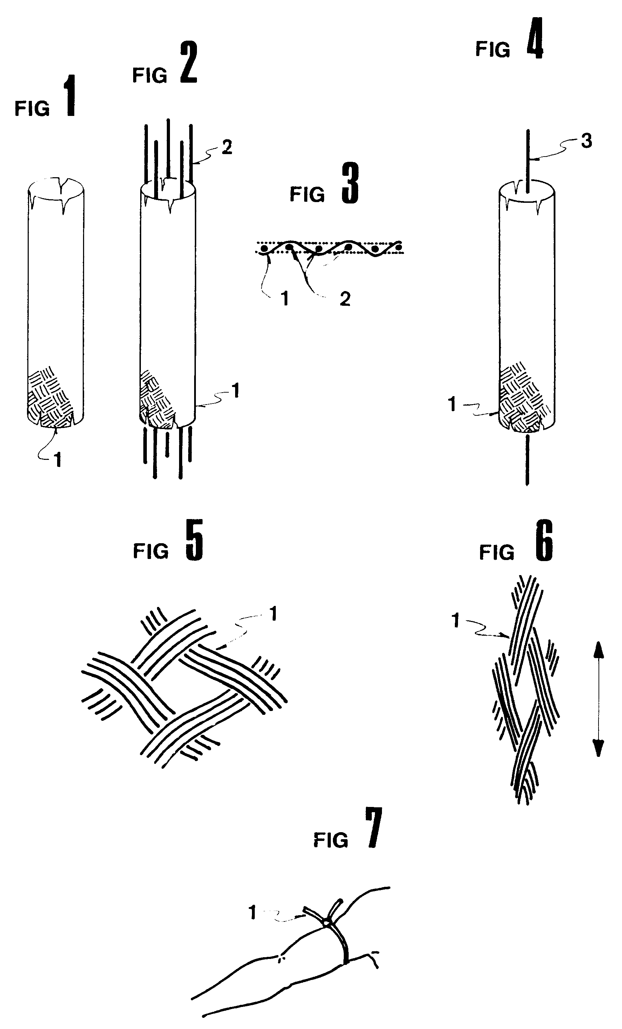

2 indicates the elastic component consisting, as in figure 2, of multiple elastic fibres inserted into the mesh of the fibrous component.

3 indicates an elastic component consisting of an elastic system inserted in a central position with respect to the tubular component as in Figure 4.

The described tubular structure, with or without elastic filaments, gives to the tourniquet, according to this invention, adequate elasticity, equal to that of rubber tourniquets.

The behaviour of the tubular structure under stretch is illustrated by the comparison between the situation in fig.5 (unstretched condition) and the one of fig.6 (stretched condition).

Said structure under stretch allows a considerable elongation. The structure allows in any case a return to the starting dimension, thanks also to the elastic filaments.

Obviously, the ends of the tourniquet are sealed or treated in such a way to avoid unravelling.

From the above description it is evident how the invention brings about the desired results.

In detail, it provides a tourniquet of little cost to be employed for a single use, with the additional advantage that it can be destroyed by incineration without polluting the atmosphere.

## Claims

1. Disposable tourniquet to be employed for a single use comprising a ribbon consisting of diagonally interwoven thread or tapes (1) and an elastic component, characterized in that said ribbon forms a tubular component and said elastic component consists of multiple elastic filaments (2) inserted into the mesh of said threads or tapes or of an elastic filament (3) inserted in an axial position with respect to the tubular component.

2. Disposable tourniquet as claimed in claim 1, characterized in that said threads or tapes (1) are of cellulose paper.

## Patentansprüche

1. Einmal-Adern-Abbindevorrichtung mit einem Band, das aus diagonal verwebten Fäden oder Bändern (1) besteht, sowie mit einem elastischen Teil, dadurch **gekennzeichnet,** daß das Band ein schlauchförmiges Teil bildet und daß das elastische Teil aus mehreren elastischen Fäden (2) besteht, die in das Netz der Fäden oder Bänder eingeschoben sind, oder aus einem elastischen Faden (3), der in bezug auf das schlauchförmige Teil in eine axiale Stellung eingeschoben ist.

2. Einmal-Adern-Abbindevorrichtung nach Anspruch 1, dadurch **gekennzeichnet,** daß die Fäden oder Bänder (1) aus Zellulose-Papier bestehen.

## Revendications

1. Tourniquet jetable à usage unique comprenant un ruban consistant en fils ou bandes (1) brochés en diagonale, et un composant élastique, caractérisé en ce que ledit ruban forme un composant tubulaire et en ce que ledit composant élastique est constitué par de multiples filaments élastiques (2) insérés dans les mailles desdits fils ou bandes ou d'un filament élastique (3) inséré en position axiale par rapport au composant tubulaire.

2. Tourniquet jetable selon la revendication 1, caractérisé en ce que lesdits fils ou rubans (1) sont en papier cellulosique.
